# EUROPEAN PATENT APPLICATION

(11) **EP 2 263 625 A1**
(43) Date of publication of application: **22.12.2010**
(21) Application number: 09713140.3
(22) Date of filing: 18.02.2009
(51) Int. Cl.: A61F 13/15, A61F 13/49, A61F 13/494

(54) **ABSORBENT ARTICLE**

(30) Priority: 19.02.2008 JP 2008037919; 19.02.2008 JP 2008037922; 06.02.2009 JP 2009026246; 06.02.2009 JP 2009026255
(71) Applicant: Unicharm Corporation, Ehime 799-0111 (JP)
(72) Inventor: OKU, Tomomi, Kagawa 769-1602 (JP); SAKAGUCHI, Satoru, Kagawa 769-1602 (JP)
(74) Representative: Kensett, John Hinton
(86) International application number: PCT/JP2009/052737
(87) International publication number: WO 2009/104626

(57) **Abstract**

An absorptive article according to the present invention includes: a main absorber 2 including at least a liquid-permeable top sheet 3, liquid-impermeable back sheet 4, and an absorber 5 disposed between the top sheet and the back sheet, absorbing and retaining fluid, and including a back-side region S1, a crotch region S2, and a belly-side region S3 arranged in the longitudinal direction L from the back side Ba to the belly side Fr in a state of wearing; and a left-and-right pair of side-flap portions 10 provided on both sides in the width direction W of the main absorber 2. A belly-side stretched region 15 extended in the around-the-waist direction provided in the belly-side region S3 of each side-flap portion 10.

## Description

### TECHNICAL FIELD

The present invention relates to an absorptive article such as diaper.

### BACKGROUND ART

There has widely been known a diaper including a main absorber composed of a liquid-permeable top sheet, a liquid-impermeable back sheet, and an absorber (see Patent Document 1, for example).

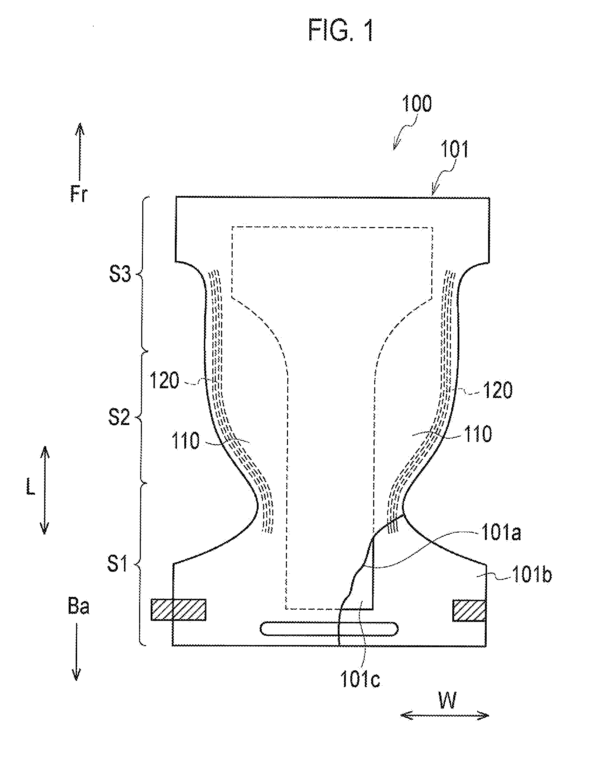

More specifically, a diaper 100, as illustrated in FIG. 1, has a main absorber 101 composed of a liquid-permeable top sheet 101a, a liquid-impermeable back sheet 101b, and an absorber 101c disposed between the sheets 101a, 101b. The absorber 101c absorbs and retains fluid.

The main absorber 101 includes a back-side region S1, a crotch region S2, and a belly-side region S3 located in the longitudinal direction L from the back-side Ba towards the belly side Fr, and covers the range from the back-side Ba to the belly side Fr of the user while locating the crotch in the center. On both sides in the width direction W of the main absorber 101, there are provided a left-and-right pair of side-flap portions 110 formed by extending the top sheet 101a and the back sheet 101b. It is to be understood that the width direction W of the main absorber 101 is the direction normal to the longitudinal direction L ranging from the back-side Ba to the belly side Fr in plan view. An elastic member 120 stretched in the longitudinal direction L is provided with each side-flap portion 110.

A pair of elastic members 120 are provided in the longitudinal direction L of the main absorber 101 in a curved pattern, rather than in a straight pattern. Accordingly, the distance between the pair of elastic members 120 is set wider in the belly-side region S3 than in the back-side region S1.

In the conventional example, the diaper 100 is excellent in the wearability, because the diaper 100 can fit to the groin of the user who wears it. The diaper 100 can form a space between the belly side Fr thereof and the user, and the space can temporarily catch urine, to thereby prevent leakage of urine to the utmost.

Another known diaper of this type is disclosed in Patent Document 2. More specifically, as illustrated in FIG. 2, a diaper 100A has, similarly to as the diaper 100, a main absorber 101A composed of a liquid-permeable top sheet 101d, a liquid-impermeable back sheet 101e, and an absorber 101f disposed between the sheets 101d, 101e.

The configuration is different from that of the diaper 100, in that the distance between a pair of elastic members 120A is set larger in the back-side region S1 than in the belly-side region S3.

In this conventional example, the diaper 100A is excellent in the wearability, because the diaper 100A can fit to, just as embracing, the hip of the user who wears it.
Patent Document 1: H11-47189 (P. 2-3, FIG. 1)
Patent Document 2: H10-243961 (P. 3, FIG. 1)

### DISCLOSURE OF THE INVENTION

Now, the conventional diaper 100 (diaper 100A) is curved so as to make the distance between the left-and-right pair of elastic members 120 (elastic members 120A) differed among the back-side region S1, the crotch region S2, and the belly-side region S3. For this reason, it may be necessary to cut the top sheet 101a (top sheet 101d) and the back sheet 101b (back sheet 101e) in a curved pattern, in order to form the side-flap portions 110 (side-flap portions 110A). As a consequence, the materials may be wasted in the process of cutting the top sheet 101a (top sheet 101d) and back sheet 101b (back sheet 101e) in a curved pattern.

In addition, the curved arrangement of the elastic members 120 (elastic members 120A) may complicate the process.

It is therefore an object of the present invention to provide an absorptive article allowing desirable fitness to the user, and capable of reducing material consumption and simplifying processes in the manufacturing.

An aspect of the present invention is summarized as an absorptive article (diaper 1A, for example) which includes: a main absorber (main absorber 2) including at least a liquid-permeable top sheet (top sheet 3), liquid-impermeable back sheet (back sheet 4), and an absorber (absorber 5) disposed between the top sheet and the back sheet, absorbing and retaining fluid, and including a back-side region (back-side region S1), a crotch region (crotch region S2), and a belly-side region (belly-side region S3) arranged in the longitudinal direction L from the back-side Ba to the belly side Fr in a state of wearing; and a left-and-right pair of side-flap portions (side-flap portions 10) provided on both sides in the width direction W of the main absorber. A belly-side stretched region (belly-side stretched region 15) extended in the around-the-waist direction (in the width direction W) provided in the belly-side region of each side-flap portion.

According to this aspect, the absorptive article may be fitted to the groin of the user who wears it, because the around-the-belly dimensions of the main absorber and the pair of side-flap portions can be larger than those in the other regions by the contribution of the belly-side stretched regions.

As a consequence, the absorptive article may be excellent in the wearability, and can form a space between the belly-side portions of the main absorber and the pair of the side-flap portions, and the user, wherein the space can temporarily catch urine, and can thereby prevent leakage of urine to the utmost. Since the belly-side stretched regions are formed by stretching parts of the pair of side-flap portions, so that it is no more necessary to form the belly side Fr of the side-flap portions larger than the other portions in the around-the-waist direction, or to form the edges on the belly side Fr in the around-the-waist direction in a curved pattern. Accordingly, the absorptive article now allows desirable fitness to the groin of the user who wears it, allows formation of a space between the belly side Fr and the user, and thereby reduces the material consumption, simplifies the processes, and consequently reduces the costs associated therewith.

An aspect of the present invention is summarized as an absorptive article (diaper 1E, for example) which includes: a main absorber (main absorber 2) including at least a liquid-permeable top sheet (top sheet 3), liquid-impermeable back sheet (back sheet 4), and an absorber (absorber 5) disposed between the top sheet and the back sheet, absorbing and retaining fluid, and including a back-side region (back-side region S1), a crotch region (crotch region S2), and a belly-side region (belly-side region S3) arranged in the longitudinal direction L from the back-side Ba to the belly side Fr in a state of wearing; and a left-and-right pair of side-flap portions (side-flap portions 10) provided on both sides in the width direction W of the main absorber. A back-side stretched region (back-side stretched region 14) extended in the around-the-waist direction (in the width direction W) provided in the back-side region of each side-flap portion.

According to this aspect, in a state of wearing, the dimension in the around-the-waist direction of the back side of the pair of side-flap portions expands by the contribution of the back-side stretched regions, so that the absorptive article may be fitted to the hip as if the back-side portions of the main absorber and the pair of side-flap portions embrace the hip, and may ensure excellent wearability. In addition, since the absorptive article can form a space between the main absorber and the user while embracing the hip by the back-side portions of the main absorber and the pair of side-flap portions, so that the space can temporarily catch excretion discharged therein, and can thereby prevent the leakage of excretion to the utmost. Since each back-side stretched region is formed by stretching portions of the pair of side-flap portions, it may be no more necessary to provide the back side Ba of each side-flap portion larger than the other portions in the around-the-waist direction, or to curve the edge in the around-the-waist direction on the back side Ba. Accordingly, the absorptive article now allows desirable fitness to the hip of the user so as to embrace it, and thereby reduces the material consumption, simplifies the processes, and consequently reduces the costs associated therewith.

According to the present invention, the diaper may be well fitted to the user, may be reduced in the material consumption, and may be simplified in processes in the manufacturing.

### BRIEF DESCRIPTION OF THE DRAWINGS

[Fig. 1] FIG. 1 is a plan view illustrating a diaper according to a conventional example.

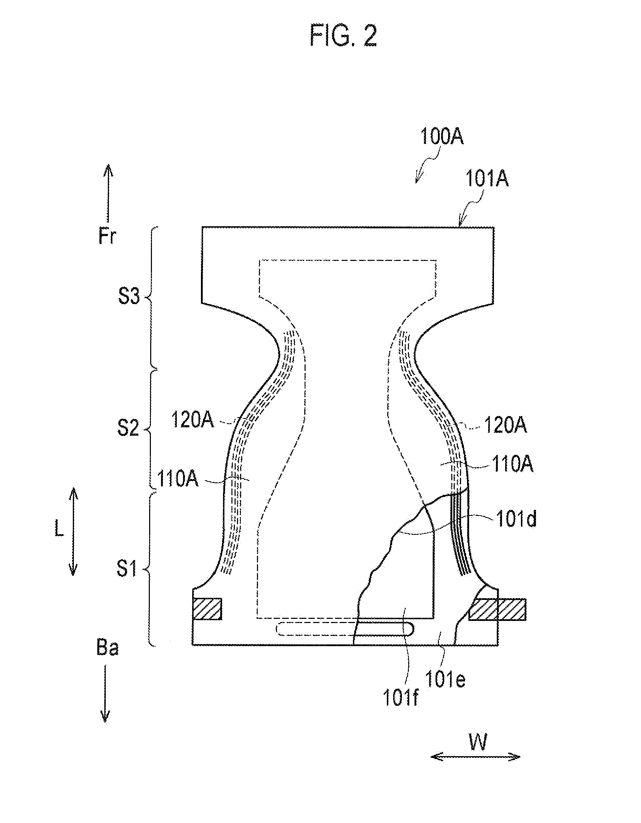

[Fig. 2] FIG. 2 is a plan view illustrating a diaper according to another conventional example.
[Fig. 3] FIG. 3 is a plan view illustrating a diaper according to a first embodiment of the present invention.
[Fig. 4] FIG. 4 is a sectional view of the diaper according to the first embodiment of the present invention, taken along line A-A in FIG. 3.
[Fig. 5] FIG. 5 is a sectional view of the diaper according to the first embodiment of the present invention, taken along line B-B in FIG. 3.
[Fig. 6] FIG. 6 is a sectional view of the diaper according to the first embodiment of the present invention in a state of wearing, taken along line B-B in FIG. 3.
[Fig. 7] FIG. 7 is a plan view illustrating the diaper according to the first embodiment of the present invention in a state of wearing.
[Fig. 8] FIG. 8 is a sectional view of an essential portion of a stretching machine manufacturing the diaper according to the first embodiment of the present invention.
[Fig. 9] FIG. 9 is a plan view of a diaper according to a second embodiment of the present invention.
[Fig. 10] FIG. 10 is a plan view of a diaper according to a third embodiment of the present invention.
[Fig. 11] FIG. 11 is a sectional view of a diaper according to a fourth embodiment of the present invention.
[Fig. 12] FIG. 12 is a plan view of a diaper according to a fifth embodiment of the present invention.
[Fig. 13] FIG. 13 is a sectional view of the diaper according to the fifth embodiment of the present invention, taken along line C-C in FIG. 12.
[Fig. 14] FIG. 14 is a sectional view of the diaper according to the fifth embodiment of the present invention, taken along line D-D in FIG. 12.
[Fig. 15] FIG. 15 is a sectional view of the diaper according to the fifth embodiment of the present invention in a state of wearing, taken along line D-D in FIG. 12.
[Fig. 16] FIG. 16 is a plan view of the diaper according to the fifth embodiment of the present invention in a state of wearing.
[Fig. 17] FIG. 17 is a plan view of a diaper according to a sixth embodiment of the present invention.
[Fig. 18] FIG. 18 is a plan view of a diaper according to a seventh embodiment of the present invention.
[Fig. 19] FIG. 19 is a sectional view of a diaper according to an eighth embodiment of the present invention.
[Fig. 20] FIG. 20 is a sectional view of a diaper according to a ninth embodiment of the present invention.

### BEST MODES FOR CARRYING OUT THE INVENTION

Next, embodiments of the present invention will be explained. In the drawings below, the same or similar constituents will be given with the same or similar reference numerals. Note that the drawings are only schematic, so that ratios of the individual dimensions may be different from actual ones.

For this reason, specific dimensions and so forth need be judged taking the explanations below into consideration. Of course some differences in dimensional relation and ratio may reside among the drawings.

### [First Embodiment]

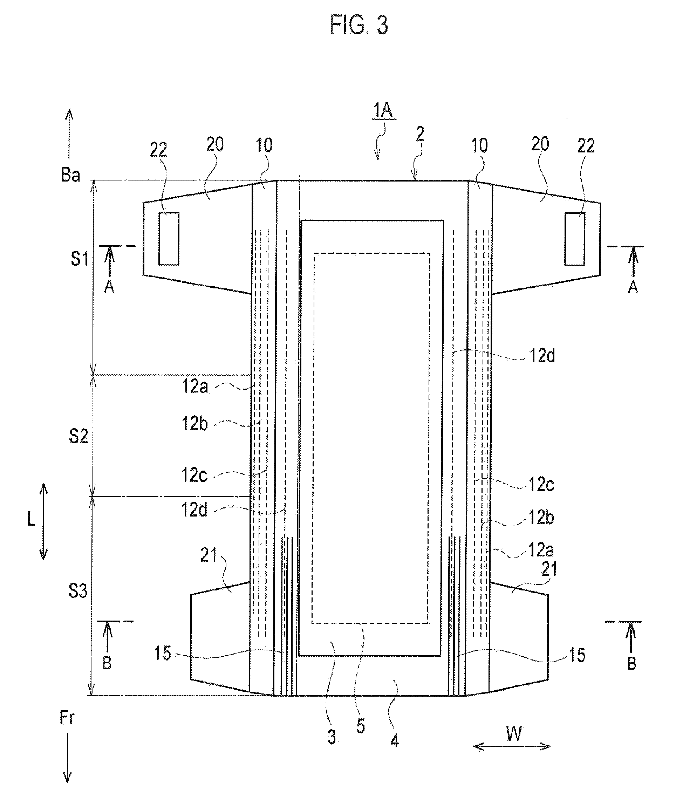

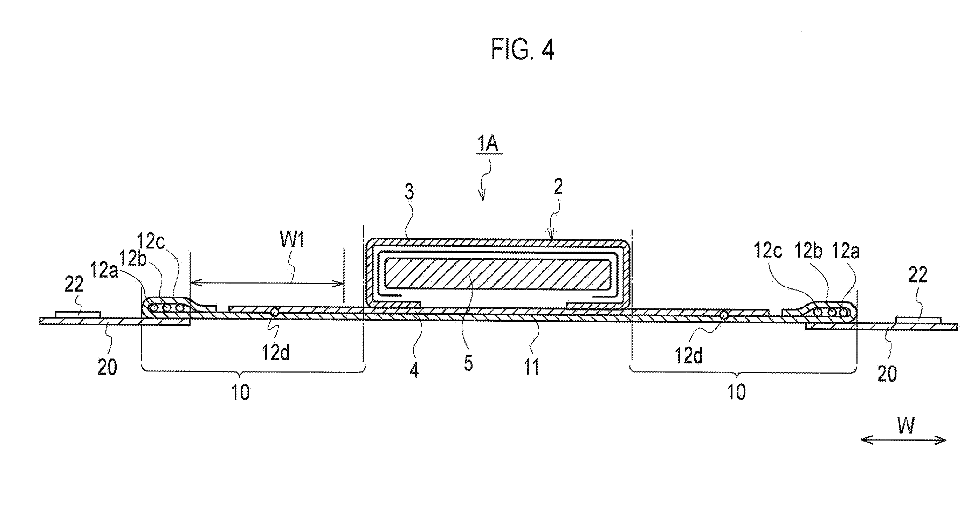

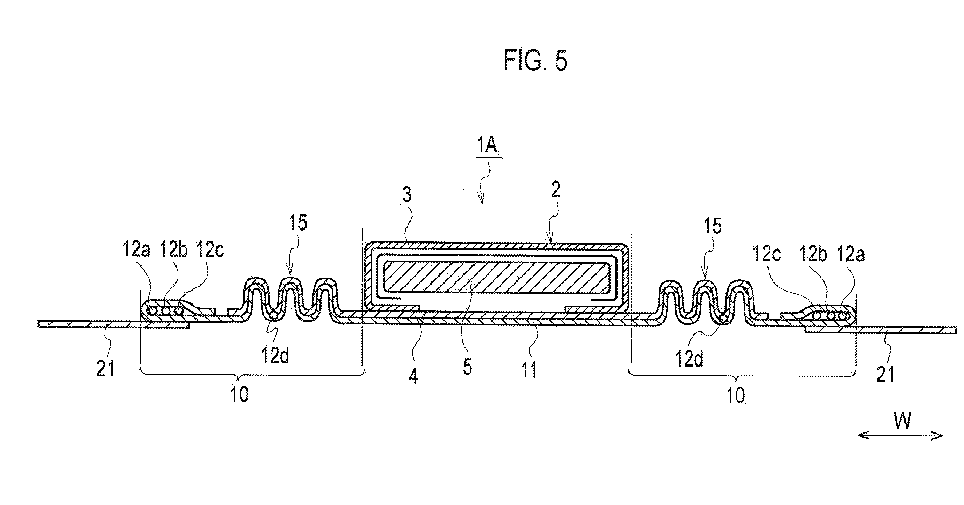

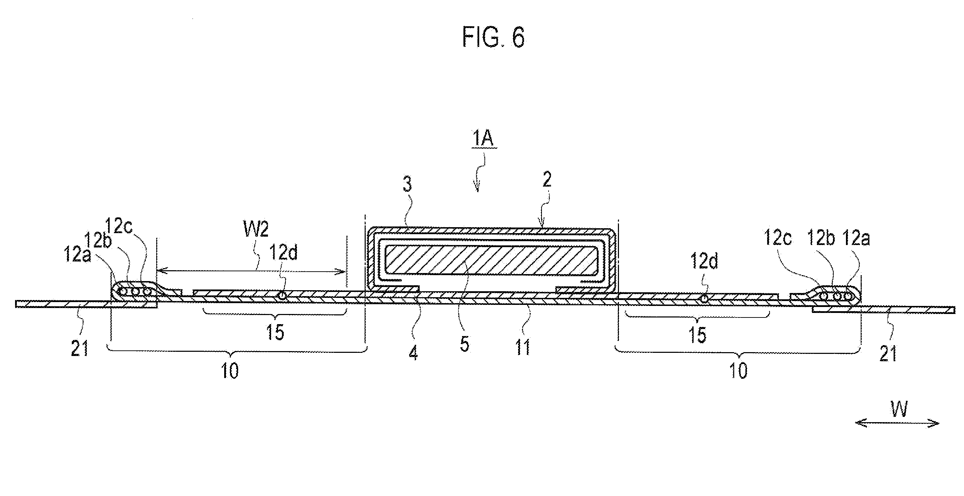

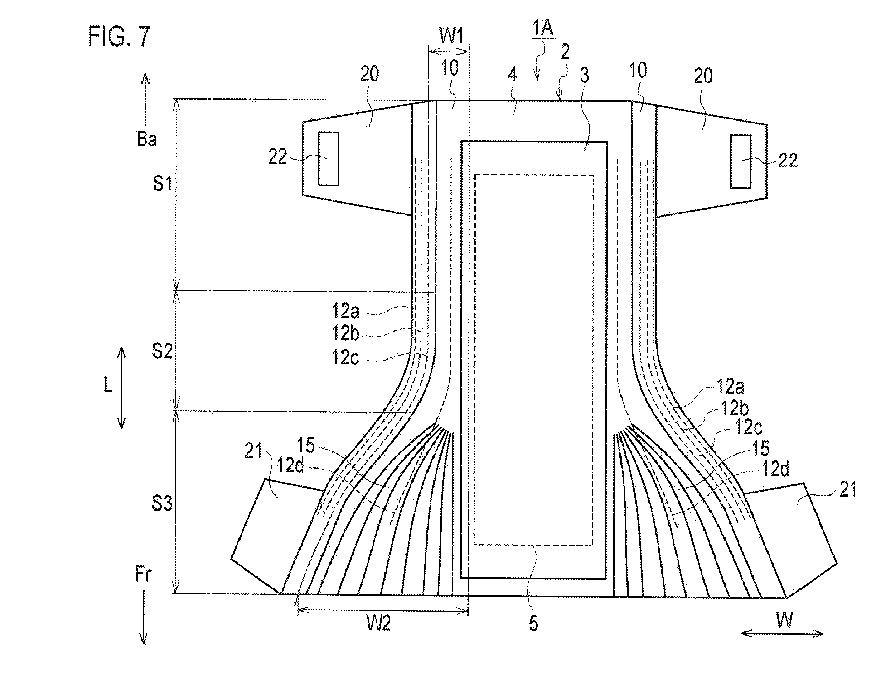

FIG. 3 to FIG. 7 illustrate a diaper 1A according to a first embodiment of the present invention. FIG. 3 is a plan view of the diaper. FIG. 4 is a sectional view taken along line A-A in FIG. 1. FIG. 5 is a sectional view taken along line B-B in FIG. 3. FIG. 6 is a sectional view of a state of wearing. FIG. 7 is a plan view of a state of wearing.

As illustrated in FIG. 3, the diaper 1A in which a back side Ba and a belly side Fr are defined and a back-side region S1, a crotch region, and a blly-side region S3 are sequentially provided along with a longitudinal direction from the back side Ba toward the belly side Fr.

As illustrated in FIG. 3 to FIG. 5, the diaper 1A as the absorptive article has a main absorber 2, a left-and-right pair of side-flap portions 10 provided on both sides of the main absorber 2 in the width direction W thereof, a pair of back-side waist flap portions 20, and a pair of belly-side waist flap portions 21.

More specifically, the back-side waist flap portions 20 are provided on both sides, in the width direction W, of a pair of side-flap portions 10 on the back side Ba thereof. The belly-side waist flap portions 21 are provided on both sides, in the width direction W, of a pair of side-flap portions 10 on the belly side Fr thereof.

The main absorber 2 is composed of at least a liquid-permeable top sheet 3, a liquid-impermeable back sheet 4, and an absorber 5 disposed between the top sheet 3 and the back sheet 4, and absorbing and retaining fluid. The main absorber 2 includes a back-side region S1, a crotch region S2, and a belly-side region S3 arranged in the longitudinal direction L from the back side Ba to the belly side Fr. The main absorber 2 covers the back side Ba, the crotch side and the belly side Fr of the user.

Both edge portions, in the width direction W, of the top sheet 3 are folded so as to wrap the absorber 5. The folded portions of the top sheet 3 and the absorber 5 are bonded to the back sheet 4, using a glue such as hotmelt adhesive. The top sheet 3 is composed of a hydrophilic non-woven fabric, woven fabric, perforated plastic film, hydrophobic non-woven fabric having perforations, or a perforated stacked non-woven fabric having a structure of (spun-bond layer)/(melt-blown layer)/(spun-bond layer), referred to as SMS hereinafter, having a basis weight of 10 g/m².

The back sheet 4 is composed of a non-ventilative film having a basis weight of 23.5g/m². The back sheet 4 may be formed also by a moisture-permeable or moisture-impermeable film, or sheet obtained by bonding a non-woven fabric to this sort of film.

The absorber 5 is a laminate having a fluid absorbing and retaining characteristics. More specifically, the absorber 5 is formed by mixing an absorptive pulp and a super absorbent polymer.

A pair of side-flap portions 10 are configured by the back sheet 4 extended from both sides of the main absorber 2 in the width direction W, and a liquid-impermeable exterior sheet 11 stacked on the back surfaces of the main absorber 2 and the back sheet 4. The back sheet 4 and the exterior sheet 11 are bonded using a glue such as hotmelt adhesive. The exterior sheet 11 is formed to have the length in the width direction W longer than that of the back sheet 4.

Over the range of each side-flap portion 10, including the crotch region S2, and portions of the back-side region S1 and the belly-side region S3 centered round the crotch region S2, there are provided four rows of elastic members 12a to 12d stretched in the longitudinal direction L.

Among four rows of elastic members, three outer rows of elastic members 12a to 12c are provided to both side portions of the exterior sheet 11 which is formed longer in the width direction W than the back sheet 4. Among four rows of elastic members, the innermost elastic member 12d is provided nearly at the center between each side edge, in the width direction W, of the main absorber 2 and the outermost elastic member 12a among four rows of elastic members. The elastic member 12d is provided between the exterior sheet 11 and the back sheet 4. The individual elastic members 12a to 12d are typically composed of polyurethane-base elastic yarn.

In the belly-side region S3 of each side-flap portion 10, and between the main absorber 2 and the outermost elastic member 12a, there is provided a belly-side stretched region 15 stretched in the around-the-waist direction (in the width direction W). More specifically, Each belly-side stretched region 15 is provided between the side edge of the main absorber 2 and to an inner-side portion close to the elastic member 12c arranged most closely to the main absorber 2 among the outer three rows of elastic members 12a to 12c provided to each side-flap portion 10.

Each belly-side stretched region 15 is a region reduced in the basis weight as compared with the other regions, by stretching effected in the around-the-waist direction normal to the longitudinal direction L from the back side Ba to the belly side Fr. In each belly-side stretched region 15, dense portions (reference numeral not given) characterized by a small amount of stretching and coarse portions (reference numeral not given) characterized by a large amount of stretching are alternately provided in the width direction W of the side-flap portion 10. Each belly-side stretched region 15 is stretched by a factor ranging from 1.5 to 3.0, as compared with the width before stretching. The factor of stretching of each belly-side stretched region 15 smaller than 1.5 may result in only a small height of rise-up of the side-flap portions 10, so that the elastic members 12a, 12b, 12c may undesirably fail in establishing close contact with the skin. On the other hand, the factor of stretching of each belly-side stretched region 15 exceeding 3.0 may heavily damage the material due to stretching, and may raise a risk of fractures of the materials, such as breakage and piercing. Accordingly, by adjusting the factor of stretching of each belly-side stretched region 15 within the range from 1.5 to 3.0, a space, or so-called pocket, may surely be formed between the main absorber 2 and the skin, without damaging the materials.

Each belly-side stretched region 15 has a transparency allowing therethrough visual recognition of internal excretion from the outside, in the state of wearing. More specifically, the transmissivity of each belly-side stretched region 15 is such as allowing seeing-through of the internal from the external. The transmissivity of light elevates by 3% or more by stretching. The internal excretion may visually be recognized from the external, if the transmissivity of light is 61% or larger (measured conforming to JIS K7105).

Each belly-side stretched region 15 in this embodiment is provided almost over the entire range between the main absorber 2 and the outermost elastic member 12a, in the width direction W of each side-flap portion 10. Each belly-side stretched region 15 is provided over the entire range of the belly-side region S3 so as to reach the edge of the belly-side region S3, in the longitudinal direction L of the side-flap portion 10. Each belly-side stretched region 15, in this embodiment, is not extended up to the crotch region S2 nor to the back-side region S1. A specific method of producing the belly-side stretched regions 15 will be detailed later.

The waist flap portion is configured by a pair of back-side waist flap portions 20 and a pair of belly-side waist flap portions 21. The pair of back-side waist flap portions 20 are fixed on one end thereof to the back-side region S1 of the pair of side-flap portions 10. To the end opposite to one end of each back-side waist flap portion 20, a binding 22 is provided.

Each binding 22 may be a hook-and-loop fastener making use of mechanical force of binding, or an adhesive tape making use of adhesive force. For the case where the bindings 22 are hook-and-loop fasteners, a material composing the belly-side waist flap portions 21 needs be a sheet material having a non-woven fabric at least on the surface thereof.

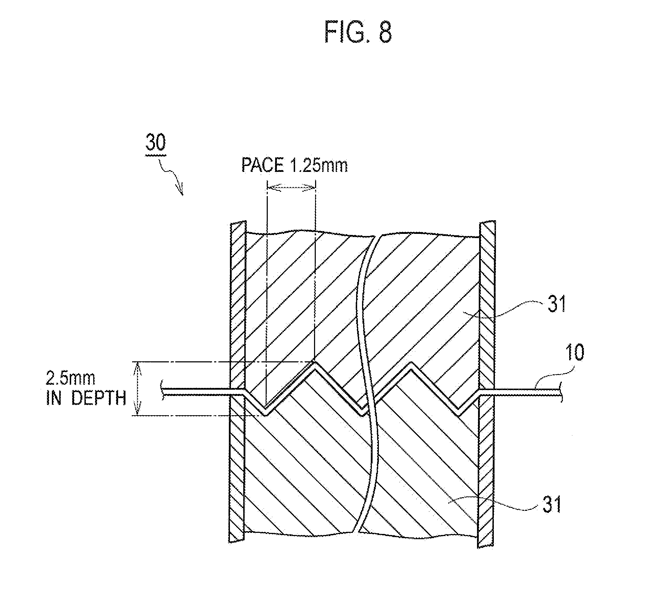

Next, a method of producing the belly-side stretched regions 15 will be briefed, referring to FIG. 8. FIG. 8 is a sectional view of a stretching machine.

The side-flap portions 10 are obtained by coating a heat-sensitive adhesive (for example, hotmelt adhesive, abbreviated as HMA hereinafter) over a 15-mm-diameter area, so as to adjust the basis weight to 5 g/m² using a spiral spray, and bonding the back sheet 4 and the exterior sheet 11.

The back sheet 4 herein is a non-ventilative film having a basis weigh of 23.5 g/m². The exterior sheet 11 is an SMS having a basis weight of 13 g/m².

A stretching machine 30 forming the belly-side stretched regions 15 has, as illustrated in FIG. 8, a pair of stretching blade array portions 31 opposed and engageable with each other. Each stretching blade array portion 31 has a depth of blade of 2.5 mm, a pitch of a pair of blades of 1.25 mm, and the number of blades of 17. The stretching machine 30 performs stretching while setting the temperature of the stretching blade array portions 31 to 100°C. By the stretching, the belly-side stretched regions 15 may be stretched by a factor of 1.8 as compared with the width before the stretching.

By stretching using the stretching machine 30, the dense portions characterized by a small amount of stretching, and coarse portions characterized by a larger amount of stretching are provided to each belly-side stretched region 15, alternatively in the width direction W (around-the-waist direction) of the side-flap portions 10.

For the case where the elastic members are arranged in the side-flap portions 10, the width of the belly-side stretched regions 15 to be stretched is preferably adjusted so as to make width W1 (FIGs. 4 and 7) fall in the range of 1.5 to 3.0 times as large as width W2 (FIGS. 6 and 7).

Now, width W1 is defined as the width measured from the side edge of the main absorber 2 at the narrowest portion of each side-flap portion 10 stretched in the width direction W, to an inner-side portion close to the elastic member 12c arranged most closely to the main absorber 2 among the outer three rows of elastic members 12a to 12c provided to each side-flap portion 10. In short, width W1 is a reference width of the side-flap portion 10, not extended by stretching in the around-the-waist direction.

On the other hand, width W2 is defined as the width measured from the side edge of the main absorber 2 at the widest portion of each side-flap portion 10 stretched in the width direction W, to an inner-side portion close to the elastic member 12c.

Now, if the elastic member 12c isn't provided to each side-flap portion 10 at the widest portion of each side-flap portion 10, as shown in this embodiment. In this case, width W2 is defined as the width measured from the side edge of the main absorber 2 at the widest portion of each side-flap portion 10 stretched in the width direction W, to an assumed line of the elastic member 12c. The assumed line is extended in the longitudinal direction L. In short, width W2 is a reference width of the side-flap portion 10 extended by stretching in the around-the-waist direction.

If width W2 is smaller than 1.5 times of width W1, the amount of stretching of the belly-side stretched region 15, reduced in the basis weight by stretching, will be too small, so that the distance between the side edge of the main absorber 2 and the position where the elastic members are arranged in the side-flap portion 10 may be shortened, and a space allowed for retaining urine effluent form the absorber 5 may be narrowed. As a consequence, excretion may flood over the portion of the side-flap portion 10 where the elastic members are arranged, to leak out from the diaper 1A.

If the width W2 exceeding 3.0 times of width W1, the amount of stretching of the belly-side stretched region 15, reduced in the basis weight by stretching, will be too large, so that the material may be more susceptible to damage by stretching, and may raise a larger risk of causing fractures such as breakage and piercing. As a consequence, fracture of the material may result in leakage of urine from the fractured portion.

Also for the case where the elastic members 12a to 12d are not arranged to the side-flap portions 10, it may be preferable, based on the same reason described in the above, to adjust the largest width to 1.5 to 3.0 times as large as the smallest width, while assuming the distance between the side edge of the main absorber 2 and the outer edge of each side-flap portion 10, when the side-flap portions 10 are stretched in the width direction W, as a reference width.

When thus-configured diaper 1A is worn by the user, the outer periphery of the individual side-flap portions 10 come into close contact with the leg-opening portion of the user, as a result of shrinkage of the individual elastic members 12a to 12d. In addition, as illustrated in FIG. 7, the belly-side region S3 having the belly-side stretched region 15 formed therein may expand in the width direction W in the state of wearing, to a degree correspondent to reduction in the basis weight by stretching.

On the other hand, the back-side region S1, which remains unstretched, will not expand beyond its intrinsic width, even if the back-side region S1 is tried to extend in the width direction W in a state of wearing.

Accordingly, in a state of wearing, the diaper 1A may be fitted to the body, widened in the belly-side region S3 than in the back-side region S1. The same will apply also to the distance between the elastic members 12a to 12d, wherein the diaper 1A worn by the user may be fitted to the body, while keeping the distance between the elastic members 12a to 12d longer in the belly side Fr than in the back side Ba.

Accordingly, the diaper 1A may be fitted to the groin of the user who wears it, and may be excellent in the wearability.

In addition, the diaper 1A can form a space, or so-called pocket, between the user and the belly side Fr portions of the main absorber 2 and the pair of side-flap portions 10. Therefore, the diaper 1A can temporarily catch urine by the pocket, and can thereby prevent leakage of urine to the utmost.

The belly-side stretched regions 15 are formed by stretching portions of the pair of side-flap portions 10. It is, therefore, no more necessary in the process of manufacturing the diaper 1A to form the belly side Fr of the side-flap portions 10 larger than the other portions in the around-the-waist direction, unlike the conventional method. In other words, it is no more necessary to curve the edge in the around-the-waist direction on the belly side Fr.

Accordingly, the diaper 1A now allows desirable fitness to the user, and forms a space between the belly side Fr of the main absorber 2 and the user, and thereby reduces the material consumption, simplifies the processes, and consequently reduces the costs associated therewith.

In addition, since the diaper 1A may be manufactured by arranging the elastic members 12a, 12b, 12c in a longitudinal straight line pattern in the side-flap portions 10, so that processes may be simplified. The belly-side stretched regions 15 in the side-flap portions 10 are given in an extended form by stretching in the width direction W, wherein the side-flap portions 10 before stretching may be formed using a nearly straight-formed material. Therefore, in the process of manufacturing the diaper 1A, the material will not be wasted due to punching, and thereby the processes may be simplified.

The individual side-flap portions 10, which cover the leg-opening portion near the crotch of the user, are configured by the thin and soft belly-side stretched regions 15, so that the diaper 1A may be less comfortable in wearing. The diaper 1A may be improved also in the compliance with motion of the hip. The belly-side stretched regions 15 raise an advantage of easy rise-up vertically to the main absorber 2.

The belly-side stretched regions 15, provided to reach the edge of the belly side Fr in this embodiment, may alternatively be provided so as not to reach the edge of the belly side Fr. It may, however, be better to provide the belly-side stretched regions 15 so as to reach the edge of the belly side Fr as shown in this embodiment, in view of better fitness to the user.

In this embodiment, each belly-side stretched region 15 is provided between the main absorber 2 and the outermost elastic member 12a. In other words, since each belly-side stretched region 15 is provided closer to the main absorber away from the width bisector between each side edge of the main absorber 2 and the outermost elastic member 12a, so that advantages below will arise. In the diaper 1A provided with the elastic members 12a to 12d in the pair of side-flap portions 10, it may be good enough to provide the elastic members in a straight line pattern, so that even the diaper having the elastic members may be simplified in the processes therefor, and may consequently be reduced in the cost. In addition, the diaper 1A worn by the user goes to bend at portions where difference in rigidity occurs, that is, at the boundary positions between the main absorber 2 and the individual side-flap portions 10. Since the portions in this embodiment are configured by the flexible belly-side stretched regions 15, so that the diaper 1A may be excellent in the rise-up performance.

Since the individual belly-side stretched regions 15 in this embodiment has transparency allowing therethrough visual recognition of internal excretion from the outside in a state of wearing, so that excretion in the diaper 1A may visually be confirmed from the outside through the belly-side stretched regions 15. The diaper 1A thus allows easy judgment of exchange of the diaper.

In this embodiment, each belly-side stretched region 15 has dense portions characterized by a small amount of stretching and coarse portions characterized by a larger amount of stretching, provided alternatively in the width direction W of each side-flap portion 10. Accordingly, in the process of manufacturing the diaper 1A, each belly-side stretched region 15 is configured by the dense portions and the coarse portions folded alternately, so that the outer edge of each side-flap portion 10 may be straight without causing projection. The diaper 1A may, therefore, be easy to handle typically in the processes of manufacturing and packaging of the products.

### [Second Embodiment]

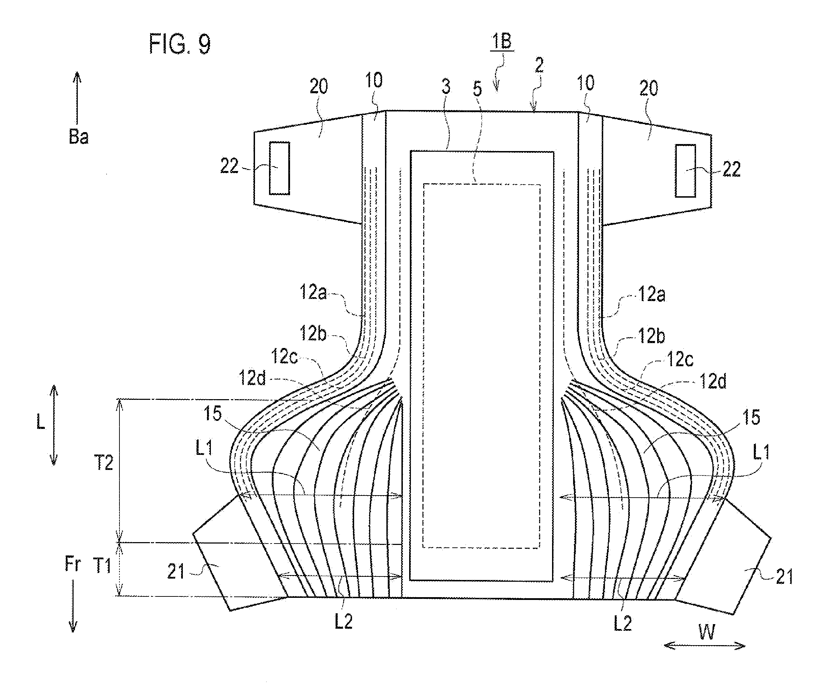

FIG. 9 is a plan view of a diaper 1B according to a second embodiment of the present invention, which illustrates a state of the diaper 1B expanded in the width for wearing.

In this embodiment, each belly-side stretched region 15 is provided so as to give different widths, rather than a uniform width, in a stretched state (in a state of wearing).

As illustrated in FIG 9, the diaper 1B according to second embodiment is different from that of the first embodiment in respect of an amount of stretching in the belly-side stretched region 15. More specifically, each belly-side stretched region 15 is provided so as to give width dimension L1 on the crotch region side T2 under stretching (in a state of wearing) larger than width dimension L2 on the belly side Fr edge side T1.

Such width dimensions may be realized by making difference in the width dimensions per se over which stretching is effected, between the crotch region side T2 and the belly side Fr edge side T1, or by making difference in the degree of stretching per unit length.

Since other aspects of the configuration are same with those in the above-described embodiment, the same reference numerals will be given in the drawing, and those explanations will be omitted.

According to this embodiment, the diaper 1B can form a large space, or so-called pocket, between the belly side Fr of the diaper 1B and the skin of the user, so that the diaper 1B can temporarily catch a large amount of urine by such large space. In other words, the diaper 1B can more reliably prevent leakage of urine.

### (Third Embodiment)

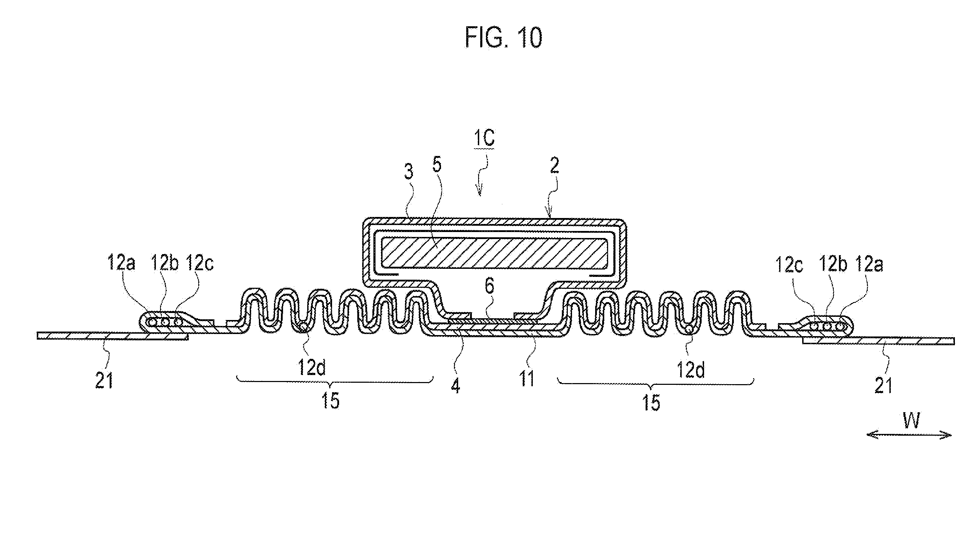

FIG. 10 is a sectional view of a diaper 1C according to a third embodiment of the present invention.

As illustrated in FIG. 10, the diaper 1C according to the third embodiment differs from the above-described first embodiment, in the configurations of the main absorber 2 and the belly-side stretched regions 15 in the individual side-flap portions 10. More specifically, the top sheet 3 and the back sheet 4 of the main absorber 2 are bonded via the bonding portion 6, only at the center portion in the width direction W, while leaving both end portions in the width direction W unbonded. Each belly-side stretched region 15 is provided so as to extend up to a region that overlaps the main absorber 2 (unbonded portion) in planar view of the diaper 1C.

Other aspects of the configuration are same with those in the above-described first embodiment, so that the explanation therefor will not be repeated, while giving instead the same reference numerals to the same components in the drawings aiming at clarity.

Since each belly-side stretched region 15 in this embodiment is provided so as to extend up to a region that overlaps the main absorber 2, the belly-side stretched region 15 may be made larger in the width-wide dimension thereof. Therefore, the diaper 1C can form a space, or so-called pocket, having a large depth on the belly side Fr thereof.

### (Fourth Embodiment)

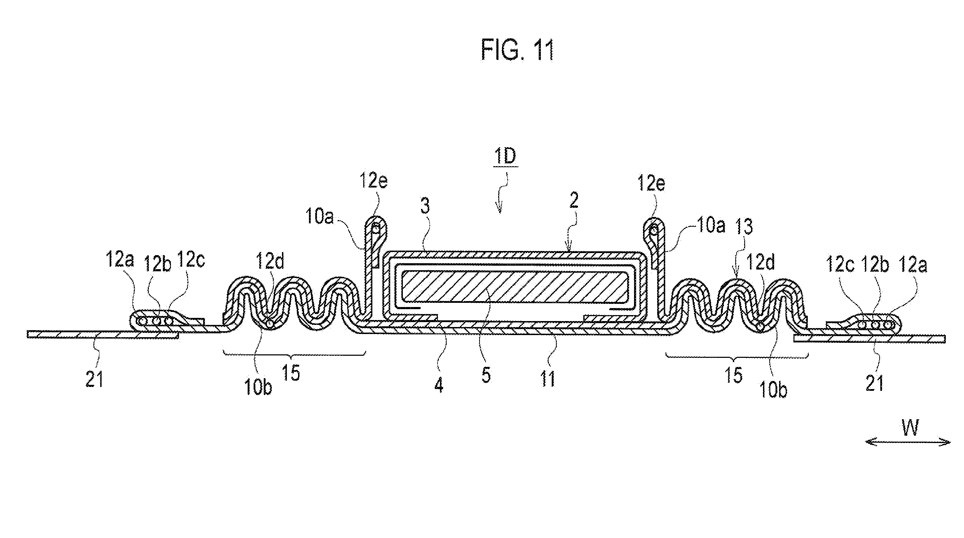

FIG. 11 is a sectional view of a diaper 1D according to a fourth embodiment of the present invention.

As illustrated in FIG. 11, the diaper 1D according to the fourth embodiment differs from the above-described first embodiment, only in the configuration of the individual side-flap portions 10. Each side-flap portion 10 branches, at a portion outside of each side-flap portion 10 in the width direction W, into two ways, so as to form an inner flap portion 10a and an outer flap portion 10b. A soft-touch sheet material is used for the inner flap portions 10a. End portions of both of the inner flap portions 10a and the outer flap portions 10b, there are provided the elastic members 12a, 12b, 12c and 12e stretched. As a consequence, when the diaper 1D is worn by the user, the side-flap portions 10 doubly come into contact with the leg-opening portion, so that the diaper 1D may more surely prevent the side leakage. The diaper 1D is also excellent in the touch, because the inner flap portions 10a are composed of a soft-touch sheet material.

Other aspects of the configuration are same with those in the above-described first embodiment, so that the explanation therefor will not be repeated, while giving instead the same reference numerals to the same components in the drawings aiming at clarity.

Although, in the fourth embodiment, the elastic members 12a, 12b, 12c and 12e were provided to the end portions of both of the inner flap portion 10a and the outer flap portion 10b, a diaper according to a modified example of the fourth embodiment may be provided with the elastic members stretched, only to the end portion of the inner flap portion 10a. The diaper according to the modified example of the fourth embodiment is now given as so-called, inwardly-inclined, three-dimensionally gathered configuration.

### (Fifth Embodiment)

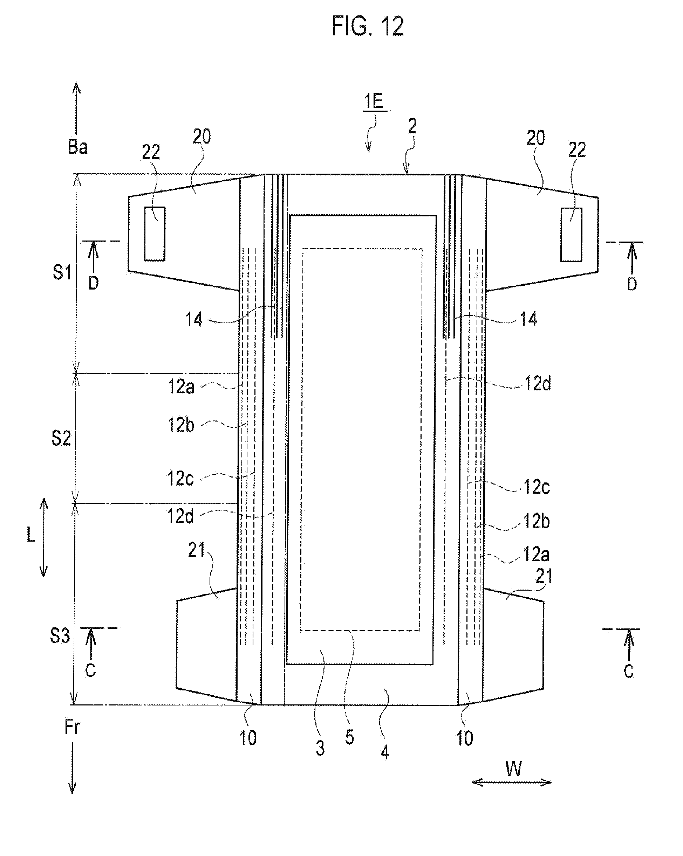

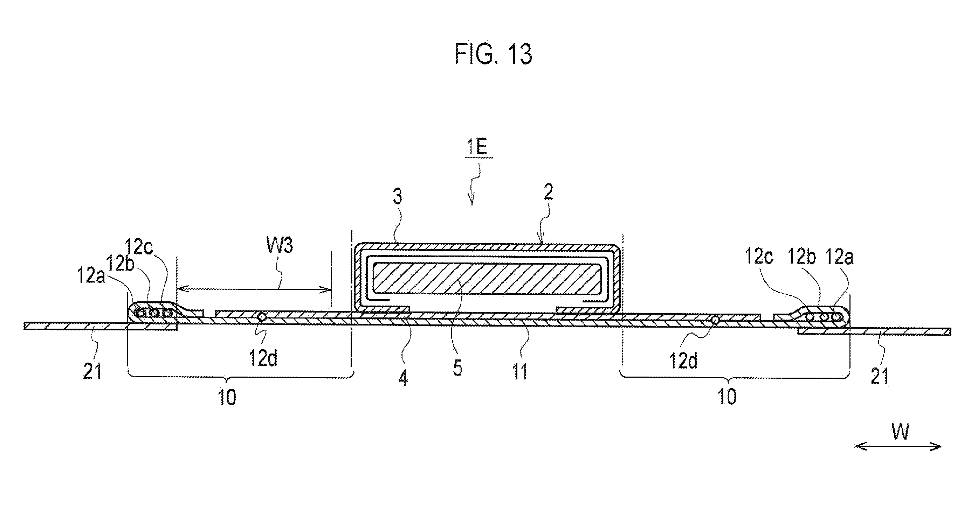

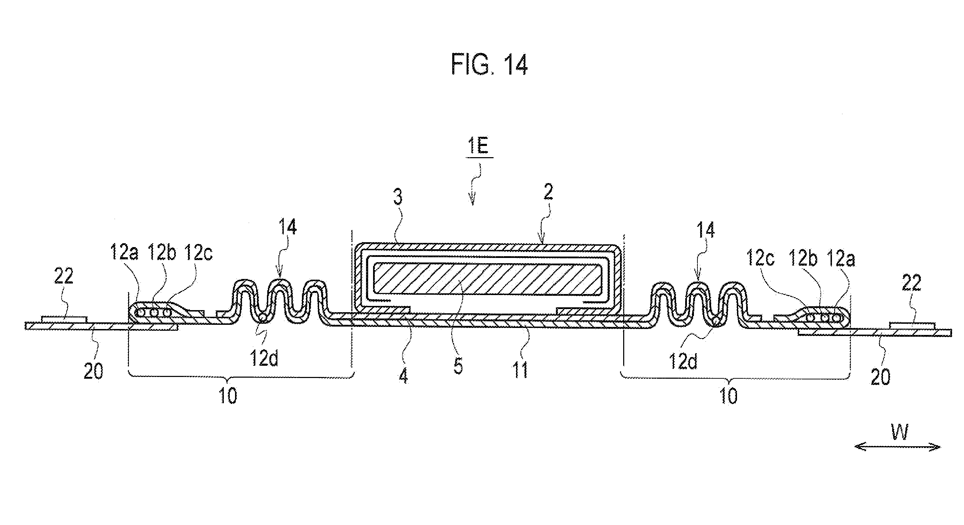

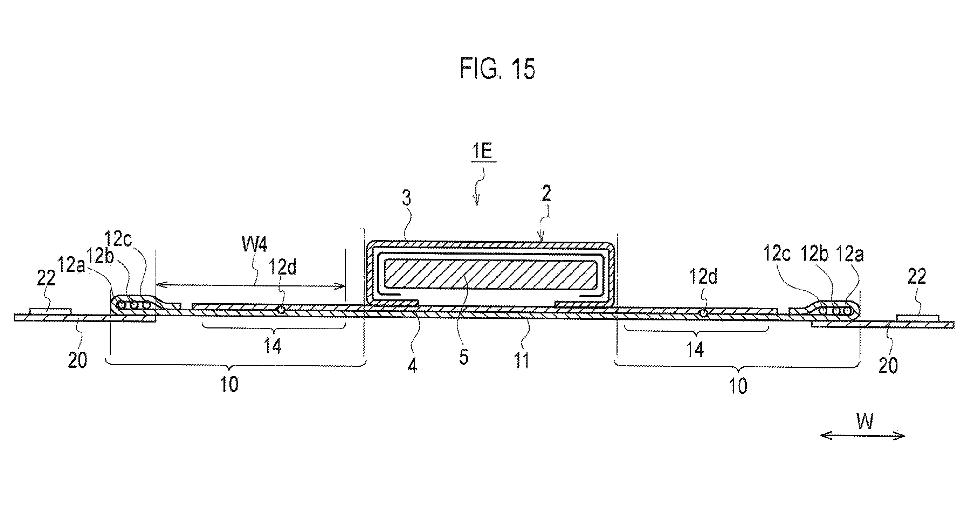

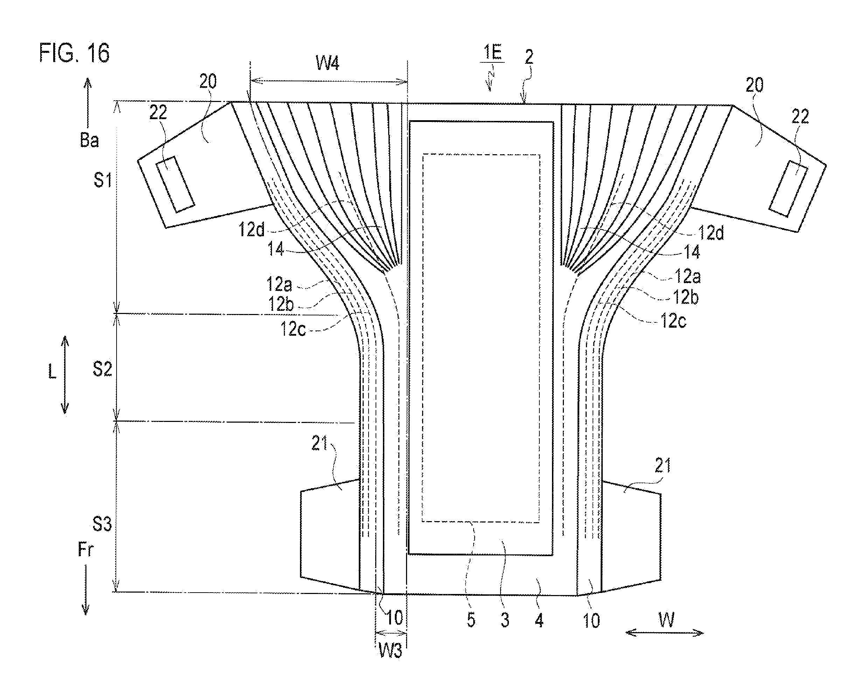

FIG. 12 to FIG. 16 illustrate a diaper 1E according to a fifth embodiment of the present invention. FIG. 12 is a plan view of the diaper. FIG. 13 is a sectional view taken along line C-C in FIG. 12. FIG. 14 is a sectional view taken along line D-D in FIG. 4. FIG. 15 is a sectional view of a state of wearing. FIG. 16 is a plan view of a state of wearing.

In the foregoing embodiments, the belly-side stretched regions 15 were provided to the belly-side region S3 of the side-flap portions. In the fifth to ninth embodiments, back-side stretched regions 14 are provided to the back-side region S1 of the side-flap portions.

Since other configurations are similar to those in the foregoing embodiments, the same reference numerals will be given in the drawing, and those explanations will be omitted.

As illustrated in FIG. 12 to FIG. 14, in the back-side region S1 of each side-flap portion 10, and between the main absorber 2 and the outermost elastic member 12a, there is provided the back-side stretched region 14 stretched in the around-the-waist direction (in the width direction W). More specifically, Each back-side stretched region 14 is provided between the side edge of the main absorber 2 and to an inner-side portion close to the elastic member 12c arranged most closely to the main absorber 2 among the outer three rows of elastic members 12a to 12c provided to each side-flap portion 10. Each back-side stretched region 14 is a region reduced in the basis weight as compared with the other regions, by stretching effected in the around-the-waist direction normal to the longitudinal direction L from the back side Ba to the belly side Fr. In each back-side stretched region 14, dense portions (reference numeral not given) characterized by a small amount of stretching and coarse portions (reference numeral not given) characterized by a large amount of stretching are alternately provided in the width direction W of the side-flap portion 10. Each back-side stretched region 14 is stretched by a factor ranging from 1.5 to 3.0, as compared with the width before stretching. The factor of stretching of each back-side stretched region 14 smaller than 1.5 may result in only a small height of rise-up of the side-flap portions 10, so that the elastic members 12a, 12b, 12c may undesirably fail in establishing close contact with the skin. On the other hand, the factor of stretching of each back-side stretched region 14 exceeding 3.0 may heavily damage the material due to stretching, and may raise a risk of fractures of the materials, such as breakage and piercing. Accordingly, by adjusting the factor of stretching of each back-side stretched region 14 within the range from 1.5 to 3.0, a space, or so-called pocket, may surely be formed without damaging the materials.

Each back-side stretched region 14 has a transparency allowing therethrough visual recognition of internal excretion from the outside, in the state of wearing. More specifically, the transmissivity of each back-side stretched region 14 is such as allowing seeing-through of the internal from the external.

Each back-side stretched region 14 in this embodiment is provided almost over the entire range between the main absorber 2 and the outermost elastic member 12a, in the width direction W of each side-flap portion 10. Each back-side stretched region 14 is provided over the entire range of the back-side region S1 so as to reach the edge of the back-side region S1, in the longitudinal direction L of the side-flap portion 10. Each back-side stretched region 14, in this embodiment, is not extended up to the crotch region S2 nor to the belly-side region S3. A specific method of producing the back-side stretched regions 14 is same as the method of producing the belly-side stretched region 15 in the above-described embodiments, so that the explanation will not be repeated.

The width of the back-side stretched regions 14 to be stretched is preferably adjusted so as to make width W3 (FIGs. 13 and 16) fall in the rang of 1.5 to 3.0 times as large as width W4 (FIGs. 15 and 16).

Now, width W3 is defined as the width measured from the side edge of the main absorber 2 at the narrowest portion of each side-flap portion 10 stretched in the width direction W, to an inner-side portion close to the elastic member 12c arranged most closely to the main absorber 2 among the outer three rows of elastic members 12a to 12c provided to each side-flap portion 10. In short, width W3 is a reference width of the side-flap portion 10, not extended by stretching in the around-the-waist direction, similar to W1.

On the other hand, width W4 is defined as the width measured from the side edge of the main absorber 2 at the widest portion of each side-flap portion 10 stretched in the width direction W, to an inner-side portion close to the elastic member 12c.

When the elastic member 12c isn't provided to each side-flap portion 10 at the widest portion of each side-flap portion 10, as shown in this embodiment, above described width W4 is defined by an assumed line extended in the longitudinal direction L. In short, the width W4 is a reference width of the side-flap portion 10 extended by stretching in the around-the-waist direction similar to W2.

If width W4 is smaller than 1.5 times of width W3, the amount of stretching of the back-side stretched regions 14, reduced in the basis weight by stretching, will be too small, so that the distance between the side edge of the main absorber 2 and the position where the elastic members are arranged in the side-flap portion 10 may be shortened, and a space allowed for retaining urine effluent form the absorber 5 may be narrowed. As a consequence, excretion may flood over the portion of the side-flap portion 10 where the elastic members are arranged, to leak out from the diaper 1E.

If the width W4 exceeding 3.0 times of width W3, the amount of stretching of the back-side stretched regions 14, reduced in the basis weight by stretching, will be too large, so that the material may be more susceptible to damage by stretching, and may raise a larger risk of causing fractures such as breakage and piercing. As a consequence, fracture of the material may result in leakage of urine from the fractured portion.

Also for the case where the elastic members 12a to 12d are not arranged to the side-flap portions 10, it may be preferable, based on the same reason described in the above, to adjust the largest width to 1.5 to 3.0 times as large as the smallest width, while assuming the distance between the side edge of the main absorber 2 and the outer edge of each side-flap portion 10, when the side-flap portions 10 are stretched in the width direction W, as a reference width.

By virtue of this relation, a large space may be formed between the user and the main absorber 2, while embracing the hip by the back side Ba portions of the main absorber 2 and the pair of side-flap portions 10. The diaper 1E may, therefore, temporarily retain a large amount of discharged excretion in such large space. In short, the diaper 1E can more reliably prevent the leakage of excretion.

Materials for the exterior sheet suitable for stretching may be any of those having a tensile rupture strength of 750% or larger. Films having a tensile rupture strength of smaller than 750% may be likely to produce pinholes during stretching. Production of pinholes may be highly causative of the leakage of excretion.

Since the transmissivity of the back-side stretched region 14 differs depending on species of sheet materials composing the side-flap portions 10, so that the amount of stretching may be adjusted depending on species of sheet materials, so as to obtain transmissivity allowing seeing-through of the inside from the outside.

When thus-configured diaper 1E is worn by the user, the outer periphery of the individual side-flap portions 10 come into close contact with the leg-opening portion of the user, as a result of shrinkage of the individual elastic members 12a to 12d. In addition, as illustrated in FIG. 16, the back-side region S1 including the back-side stretched region 14 formed therein may expand in the width direction W in the state of wearing, to a degree correspondent to reduction in the basis weight by stretching.

On the other hand, the belly-side region S3, which remains unstretched, will not expand beyond its intrinsic width, even if the belly-side region S3 is tried to extend in the width direction W in a state of wearing.

Accordingly, in a state of wearing, the diaper 1E may be fitted to the body widened more largely in the back-side region S1 than in the belly-side region S3. The same will apply also to the distance between the elastic members 12a to 12d, wherein the diaper 1E worn by the user may be fitted to the body, while keeping the distance between the elastic members 12a to 12d longer in the back side Ba than in the belly side Fr.

Accordingly, the diaper 1E may be fitted to the user, just as embracing the hip by the back side Ba thereof, and may be excellent in the wearability.

In addition, the diaper 1E can form a spatial margin between itself and the user, while embracing the hip. Therefore, even when loose stool is discharged, the diaper 1E may be able to temporarily catch the loose stool. In short, the diaper 1E can prevent the loose stool, effluent from the absorber, from immediately flooding over the elastic members 12a to 12d at the leg-opening portion, and from leaking.

Since the back-side stretched regions 14 are formed by stretching in the width direction W, so that the side-flap portions 10 before stretching may be formed using a nearly straight-formed material. Therefore, in the process of manufacturing the diaper 1E, the material will not be wasted due to punching, and thereby the processes may be simplified.

The individual side-flap portions 10, which cover the leg-opening portion near the crotch of the user, are configured by the thin and soft back-side stretched regions 14, so that the diaper 1E may be less comfortable in wearing. The diaper 1E may be improved also in the compliance with motion of the hip. The back-side stretched regions 14 raises advantages of easy rise-up vertically to the main absorber 2, and of excellent fitting to the hip without excessively tightening the outer periphery of the individual side-flap portions 10.

The back-side stretched regions 14, provided to reach the edge of the back side Ba in this embodiment, may alternatively be provided so as not to reach the edge of the back side Ba. It may, however, be better to provide the back-side stretched regions 14 so as to reach the edge of the back side Ba as shown in this embodiment, in view of better fitness quality to the user.

In this embodiment, each back-side stretched region 14 is provided between the main absorber 2 and the outermost elastic member 12a. In other words, since each back-side stretched region 14 is provided more closer to the main absorber away from the width bisector between each side edge of the main absorber 2 and the outermost elastic member 12a, so that advantages below will arise. In the diaper provided with the elastic members 12a, 12b, 12c to the pair of side-flap portions 10, it may be good enough to provide the elastic members in a straight line pattern, so that even the diaper having the elastic members may be simplified in the processes therefor, and may consequently be reduced in the cost. In addition, the diaper 1E worn by the user goes to bend at portions where difference in rigidity occurs, that is, at the boundary positions between the main absorber 2 and the individual side-flap portions 10. Since the portions in this embodiment are configured by the flexible back-side stretched regions 14, so that the diaper 1E may be excellent in the rise-up performance, and may be improved in the fitness to the hip.

Since the individual back-side stretched regions 14 in this embodiment has transparency allowing therethrough visual recognition of internal excretion from the outside in a state of wearing, so that excretion in the diaper 1E may visually be confirmed from the outside through the back-side stretched regions 14. The diaper 1E thus allows easy judgment of exchange of the diaper.

In this embodiment, each back-side stretched region 14 has dense portions characterized by a small amount of stretching and coarse portions characterized by a larger amount of stretching, provided alternatively in the width direction W of each side-flap portion 10. Accordingly, in the process of manufacturing the diaper 1E, each back-side stretched region 14 is configured by the dense portions and the coarse portions folded alternately, so that the outer edge of each side-flap portion 10 may be straight without causing projection. The diaper 1E may, therefore, be easy to handle typically in the process of manufacturing and packaging of the products.

### (Sixth Embodiment)

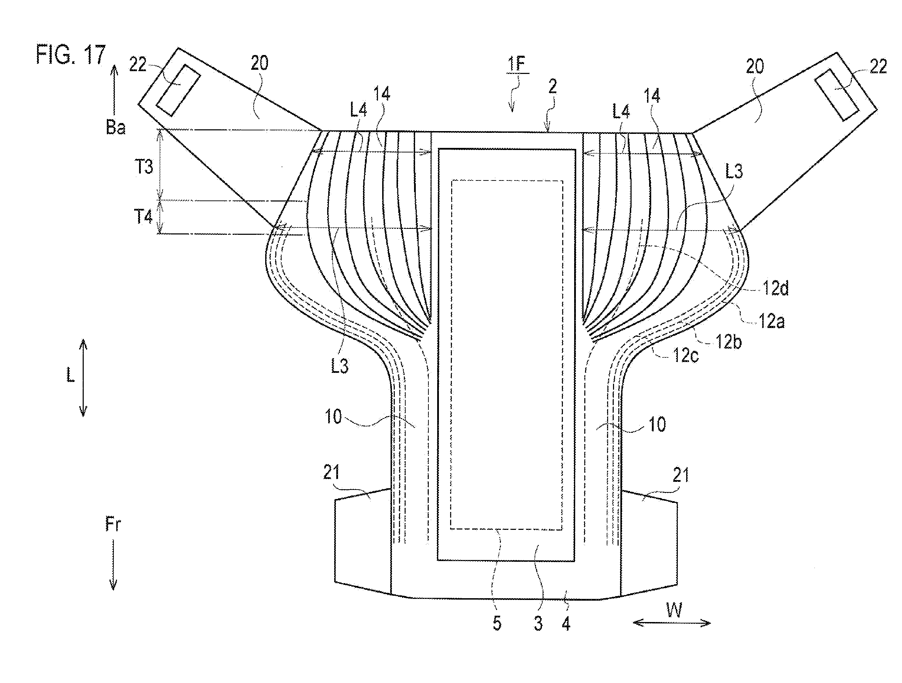

FIG. 17 is a plan view of a diaper 1F according to a sixth embodiment of the present invention, illustrating a state that the diaper 1F was expanded in the width for wearing.

As illustrated in FIG. 17, in the diaper 1F according to the sixth embodiment, the amount of stretching of the back-side stretched regions 14 is different from that in the fifth embodiment. More specifically, width dimension L3 on the crotch region side T4 in a stretched state (in a state of wearing) is made larger than the width dimension L3 on the crotch region side T4.

Since other configurations are similar to those in the fifth embodiment, the same reference numerals will be given in the drawing, so as to avoid repetitive explanations therefor.

According to this embodiment, the diaper 1F can form a large space between the hip and the main absorber 2, in a state of wearing, and can temporarily retain a large amount of excretion. In short, the diaper 1F can more reliably prevent the leakage of excretion.

### (Seventh Embodiment)

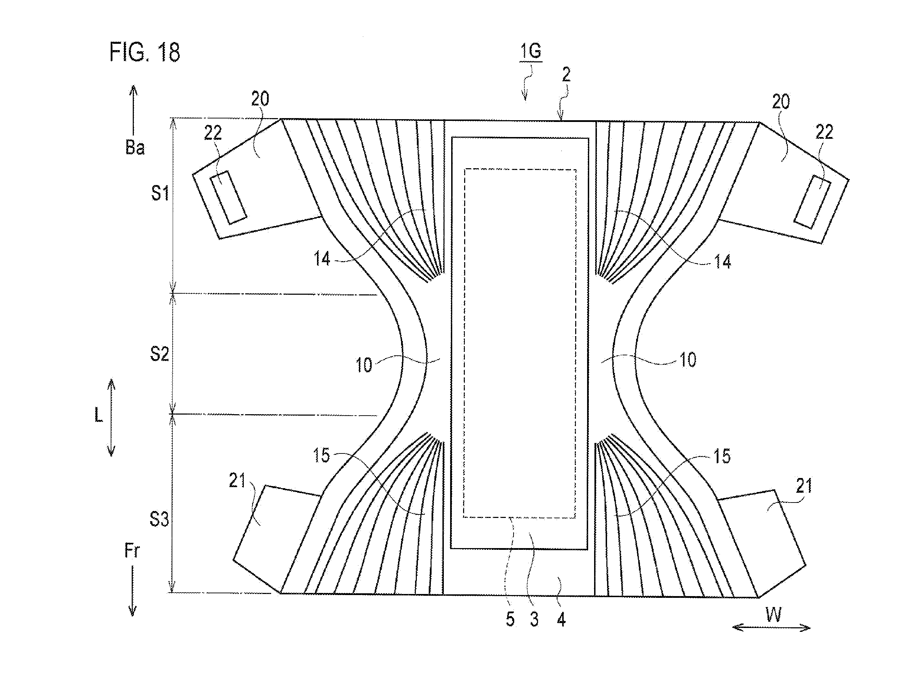

FIG. 18 is a plan view of a diaper 1G according to a seventh embodiment of the present invention.

In this embodiment, the back-side stretched regions 14 are formed in the back-side region S1 of the pair of side-flap portions 10, and the belly-side stretched regions 15 are formed in the belly side region S3. The back-side region S1 and the belly-side region S3 are therefore widened as compared with the crotch region S2, and thereby the diaper 1G can form spaces both on the back side Ba and belly side Fr. Accordingly, in the diaper 1G, the space is formed between the main absorber 2 and the user also on the belly side regions of the main absorber 2 and the pair of side-flap portions 10, while embracing the crotch portion. Therefore, the diaper 1G may temporarily retain a large amount of discharged excretion in both spaces on the back side Ba and on the belly side Fr, and can thereby prevent the leakage of excretion to the utmost.

Note that, also in the belly-side stretched regions 15, the amount of stretching may be differed between the crotch-side region and the back-side region, similarly to as in the sixth embodiment. The diaper may thus be made excellent in the wearability.

### (Eighth Embodiment)

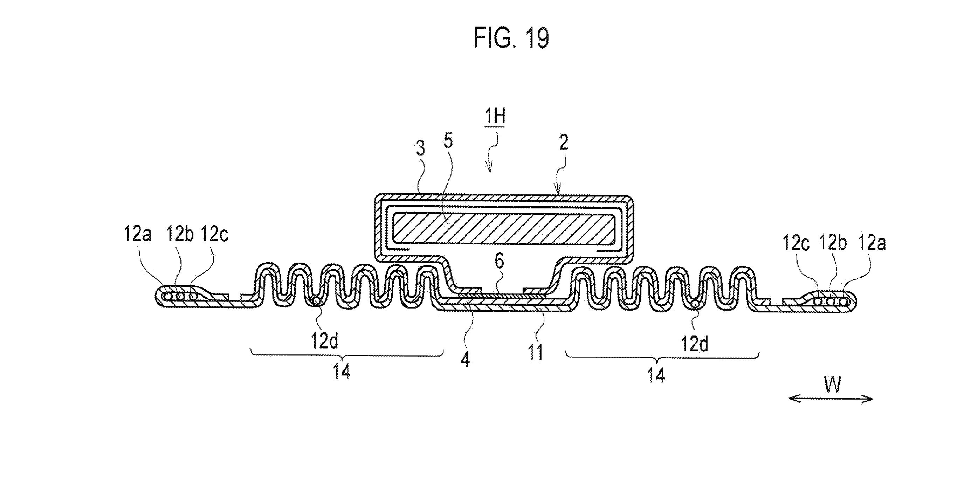

FIG. 19 is a sectional view of a diaper 1H according to an eighth embodiment of the present invention.

As illustrated in FIG. 19, the diaper 1H according to the eight embodiment is different from the fifth embodiment, in the configurations of the main absorber 2 and the back-side stretched regions 14 of the individual side-flap portions 10. More specifically, the top sheet 3 and the back sheet 4 of the main absorber 2 are bonded via the bonding portion 6, only at the center portion in the width direction W, while leaving both end portions unbonded. Each back-side stretched region 14 is provided so as to extend up to a region that overlaps the main absorber 2 (unbonded portion) in planar view of the diaper 1H.

Other aspects of the configuration are same with those in the fifth embodiment, so that the explanation therefor will not be repeated, while giving instead the same reference numerals to the same components in the drawings aiming at clarity.

Since, in this embodiment, each back-side stretched region 14 is provided so as to extend up to the region that overlaps the main absorber 2, so that the width dimension of the back-side stretched region 14 may be large enough. Accordingly, the diaper 1H may ensure a large dimension allowing embracement of the hip, and a large space, or so-called pocket, may be formed on the back side Ba of the diaper 1H.

### (Ninth Embodiment)

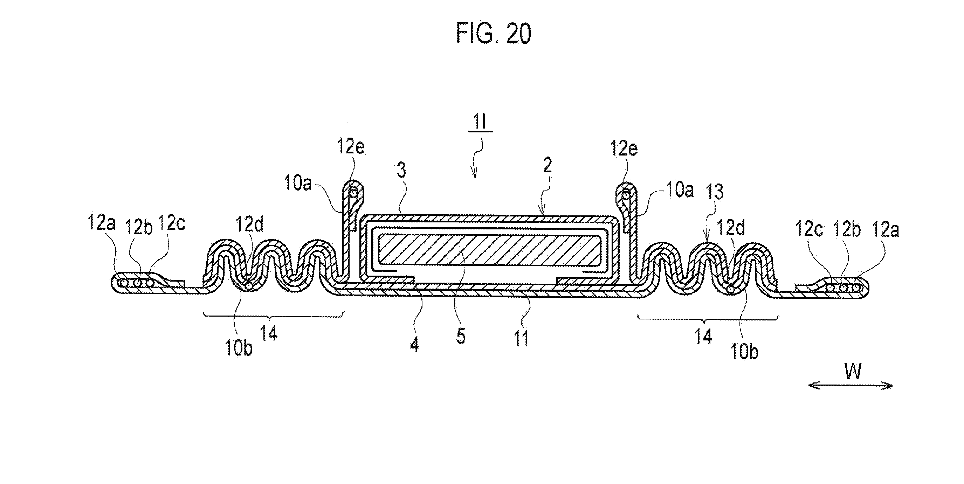

FIG. 20 is a sectional view of a diaper 1l according to a ninth embodiment of the present invention.

As illustrated in FIG. 20, the diaper 1l according to the ninth embodiment differs from that in the fifth embodiment, only in the configuration of the individual side-flap portions 10. Each side-flap portion 10 branches, at a portion outside each side-flap portion 10 in the width direction W, into two ways, so as to form an inner flap portion 10a and an outer flap portion 10b. A soft-touch sheet material is used for the inner flap portions 10a. In the end portions of both of the inner flap portions 10a and the outer flap portions 10b, there are provided the elastic members 12a, 12b, 12c and 12e stretched. As a consequence, when the diaper 1l is worn by the user, the side-flap portions 10 doubly come into contact with the leg-opening portion, so that the diaper 1l may more surely prevent the side leakage. The diaper 1l is also excellent in the touch, because the inner flap portions 10a are composed of a soft-touch sheet material. Other aspects of the configuration are same with those in the fifth embodiment, so that the explanation therefor will not be repeated, while giving instead the same reference numerals to the same components in the drawings aiming at clarity.

### (Modified Examples of Foregoing Embodiments, and Others)

In a diaper according to modified examples of the individual embodiments, a crotch-side stretched region may be provided to the crotch region S2 in each side-flap portions 10, and between the main absorber 2 and the outermost elastic member 12a.

The crotch-side stretched region may be formed by stretching the crotch region S2 of each side-flap portion 10 in the width direction W. By providing the crotch-side stretched regions in this way, the diaper according to the modified examples may bring the outer periphery of the individual side-flap portions 10 into close contact with the leg-opening portion of the user who wears it, as a result of shrinkage of the individual elastic members.

Since the expandable width dimension of the crotch region S2 of the individual side-flap portions 10 is set larger in the crotch region S2 by the contribution of the crotch-side stretched region, than in the back-side region S1 and the belly-side region S3, so that a space, or so-called pocket, is formed in the crotch side of the user. The diaper according to the modified examples may, therefore, form the pocket on the crotch side, without providing the folded portions to the individual side-flap portions 10, without curving the edge of the individual side-flap portions 10, or without curving the elastic members along the curved profile of the edge in the width direction W of the individual side-flap portion 10. Therefore in the process of manufacturing the diaper according to the modified examples, the material consumption may be reduced, processes may be simplified, and the cost associated therewith may be reduced.

While the belly-side stretched regions 15 and the back-side stretched regions 14 in the present invention were formed to both of the back sheet 4 and the exterior sheet 11, the belly-side stretched regions 15 and the back-side stretched regions 14 may be formed by stretching the back sheet 4 only. In this case, consumption of the non-woven fabric may be reduced.

Alternatively, the belly-side stretched regions 15 and the back-side stretched regions 14 may be formed by stretching portions where three members, including the top sheet 3, the back sheet 4 and the exterior sheet 11, are bonded. In this case, body fluid effluent from the absorber 5 may be guided by the hydrophilic top sheet 3 back again to the absorber 5.

In the present invention, the exterior sheet 11 of the pair of side-flap portions 10 may be stacked with a fluid-impermeable sheet composed of a material different from that of the back sheet 4, and the portion where the exterior sheet 11 and the fluid-impermeable sheet are stacked may be stretched to form the belly-side stretched regions 15 and the back-side stretched regions 14. In this case, most suitable material may be disposed at every portion, such as using a sheet material suitable for stretching for the fluid-impermeable sheet, and using a ventilative film or a high-density film aimed at pinhole prevention for the back sheet 4 lying under the absorber 5.

Although in the above-described embodiments, each side-flap portion 10 was provided with four rows of elastic members 12a to 12d, the elastic members may also be provided to each side edge of the side-flap portion 10 only in a single row or two rows. The number of elastic members and position of arrangement may appropriately be modified.

Although the above embodiments explained the case where the present invention was applied to the open-type diapers, the present invention is similarly applicable also to the pants-type diapers. The present invention is applicable still also to any absorptive articles other than the diapers.

This application is based the prior Japanese Patent Application No. JP 2008-037919, filed on February 19, 2008; Japanese Patent Application No. JP 2008-037922, filed on February 19, 2008; Japanese Patent Application No. JP 2009-026246, filed on February 06, 2009; and Japanese Patent Application No. JP 2009-026255, filed on February 06, 2009 and the entire contents of which are incorporated herein by reference.

### INDUSTRIAL APPLICABILITY

As has been described above, the absorbent article according to the present invention can fit user, and is applicable to reduce material consumption and simplify processes in the manufacture.

## Claims

1. An absorptive article comprising:
a main absorber including at least a liquid-permeable top sheet, liquid-impermeable back sheet, and an absorber disposed between the top sheet and the back sheet, absorbing and retaining fluid,
and including a back-side region, a crotch region, and a belly-side region arranged in the longitudinal direction L from the back side to the belly side in a state of wearing; and
a left-and-right pair of side-flap portions provided on both sides in the width direction W of the main absorber, wherein
a belly-side stretched region extended in the around-the-waist direction provided in the belly-side region of each side-flap portion.

2. The absorptive article according to claim 1, wherein
each belly-side stretched region reaches the belly-side edge.

3. The absorptive article according to claim 1, wherein
elastic members stretched in the longitudinal direction L provided in the crotch region of each side-flap, and
each belly-side stretched region is provided between the main absorber and the outermost elastic member.

4. The absorptive article according to claim 1, wherein
a width dimension of each belly-side stretched region in a stretched state is larger on the crotch region side than on the belly-side edge side.

5. The absorptive article according to claim 1, wherein
each belly-side stretched region is extended up to a region that overlaps the main absorber.

6. The absorptive article according to claim 1, wherein
each belly-side stretched region is stretched by a factor ranging from 1.5 to 3.0.

7. An absorptive article comprising:
a main absorber including at least a liquid-permeable top sheet, liquid-impermeable back sheet, and an absorber disposed between the top sheet and the back sheet, absorbing and retaining fluid, and including a back-side region, a crotch region, and a belly-side region arranged in the longitudinal direction L from the back side to the belly side in a state of wearing; and
a left-and-right pair of side-flap portions provided on both sides in the width direction W of the main absorber, wherein
a back-side stretched region extended in the around-the-waist direction provided in the back-side region of each side-flap portion.

8. The absorptive article according to claim 7, wherein
each back-side stretched region reaches the back-side edge.

9. The absorptive article according to claim 7, wherein
elastic members stretched in the longitudinal direction L provided at least in the crotch region of each side-flap portion, and
each back-side stretched region is provided between the main absorber and the outermost elastic member.

10. The absorptive article according to claim 7, wherein
a width dimension of each back-side stretched region in a stretched state is larger on the crotch region side than on region of each side-flap portion.

11. The absorptive article according to claim 7, wherein
each back-side stretched region is extended up to a region that overlaps the main absorber.

12. The absorptive article according to claim 7, wherein
each back-side stretched region is stretched by a factor ranging from 1.5 to 3.0.

13. The absorptive article according to claim 7, wherein
a belly-side stretched region extended in the around-the-waist direction provided in the belly-side region of each side-flap portion.

14. The absorptive article according to claim 1 or 7, wherein
a crotch-side stretched region stretched in the width direction W provided between the main absorber and the outermost elastic member in the crotch region of each side-flap portion.
